# EUROPÄISCHE PATENTSCHRIFT

(11) **EP 1 286 756 B1**
(45) Veröffentlichungstag und Bekanntmachung des Hinweises auf die Patenterteilung: **18.08.2004**
(21) Anmeldenummer: 01940536.4
(22) Anmeldetag: 29.05.2001
(51) Int. Cl.: B01F 3/12, B01F 17/00

(54) **VERFAHREN ZUR HERSTELLUNG VON NANOPARTIKEL-SUSPENSIONEN**
METHOD FOR PRODUCING NANOPARTICLE SUSPENSIONS
PROCEDE POUR PRODUIRE DES SUSPENSIONS DE NANOPARTICULES

(30) Priorität: 08.06.2000 DE 10027948
(43) Veröffentlichungstag der Anmeldung: 05.03.2003
(73) Patentinhaber: Henkel Kommanditgesellschaft auf Aktien, 40589 Düsseldorf (DE)
(72) Erfinder: STALBERG, Theo, 40789 Monheim (DE); SCHRÖDER, Christine, 40593 Düsseldorf (DE); DOLHAINE, Hans, 41352 Korschenbroich (DE)
(86) Internationale Anmeldenummer: PCT/EP2001/006077
(87) Internationale Veröffentlichungsnummer: WO 2001/093996

(56) Entgegenhaltungen:
- EP-A- 0 506 197
- WO-A-00/21490
- DE-A- 19 837 191
- US-A- 5 662 932

## Beschreibung

Die Erfindung betrifft ein Verfahren zur Herstellung einer Suspension eines unzersetzt schmelzbaren Stoffes mit einem mittleren Teilchendurchmesser im Bereich von 5 bis 500 nm.

Suspensionen von Stoffen mit einer besonders geringen Teilchengröße, insbesondere einer Teilchengröße von unterhalb 1 Mikrometer, haben in der jüngeren Zeit in unterschiedlichen Bereichen vielfältige Anwendungsmöglichkeiten gefunden. Zur Herstellung feinstteiliger Suspensionen von Stoffen sind aus der Literatur unterschiedliche Verfahren bekannt. Soweit es sich um schmelzbare Stoffe handelt, haben sich dabei Verfahren bewährt, im Verlauf derer eine Schmelze der Stoffe emulgiert und anschließend abgekühlt wird.

So beschreibt die EP-B1 0 506 197 ein Verfahren zur Herstellung wäßriger Dispersionen von Lipid-Nanopartikeln, in welchem zunächst das Lipid in einer wäßrigen Phase geschmolzen wird, wobei optional Emulgatoren anwesend sein können. Anschließend wird das Gemisch aus geschmolzenem Lipid und wäßriger Phase durch intensive Durchmischung dispergiert. Dabei wird das Lipid in nanopartikuläre Tröpfchen in einem Größenbereich von 50 bis 1000 nm überführt, welche man durch Abkühlen erstarren läßt unter Ausbildung der erfindungsgemäßen Dispersion. Bei diesem Verfahren ist eine sehr intensive Durchmischung erforderlich, um die gewünschten Tröpfchen im Nanometerbereich zu erzielen. Gemäß den aufgeführten Beispielen ist eine Durchmischung mit einem Turrax-Homogenisator dabei nicht ausreichend, sondern erst durch die zusätzliche Anwendung eines Hochdruckhomogenisators vom Mikrofluidizer-Typ kann eine stabile Suspension mit einer Partikelgröße im Nanometer-Bereich erzielt werden.

Die aus dem Stand der Technik bekannten Verfahren zur Herstellung nanopartikulärer Suspensionen schmelzbarer Stoffe erfüllen nicht gleichzeitig die Anforderungen nach
a) einer auch im technischen Maßstab einfachen und kostengünstigen Durchführbarkeit, worunter insbesondere ein aus möglichst wenigen Schritten bestehendes, mit einer hohen Raum-Zeit-Ausbeute verlaufendes Verfahren zu verstehen ist, das einen minimalem technischen Aufwand erfordert,
b) einer hohen Lagerstabilität der erzeugten Suspensionen und
c) der Möglichkeit, auch konzentrierte Suspensionen herzustellen.

Aufgabe der Erfindung war es, den Mängeln des Standes der Technik abzuhelfen und ein Herstellverfahren bereitzustellen, welches diese vorstehend genannten Anforderungen erfüllt.

Die Aufgabe wird gelöst durch die Bereitstellung eines Verfahrens zur Herstellung einer Suspension eines unzersetzt schmelzbaren Stoffes mit einen mittleren Teilchendurchmesser im Bereich von 5 bis 500 nm, bei dem man
(a) eine Emulsion herstellt aus dem unzersetzt schmelzbaren Stoff, einer flüssigen Phase, in welcher der Stoff schwerlöslich ist, sowie einer wirksamen Menge mindestens eines Oberflächenmodifikationsmittels, und
(b) die resultierende Emulsion in eine Zone verminderten Druckes so entspannt, daß die Emulsion unter den Schmelzpunkt des Stoffes abgekühlt wird.

In einer bevorzugten Ausführungsform der Erfindung liegt der mittlere Teilchendurchmesser des unzersetzt schmelzbaren Stoffes in der Suspension im Bereich von 10 bis 300 nm und besonders bevorzugt zwischen 20 und 100 nm.

Die Größenangaben sind zu verstehen als Durchmesser in Richtung der größten Längenausdehnung der Teilchen. Bei der Herstellung der feinteiligen Partikel erhält man stets Teilchen mit einer Größe, die einer Verteilungskurve folgt. Zur experimentellen Bestimmung der Teilchengröße kann beispielsweise die dem Fachmann bekannte Methode der dynamischen Lichtstreuung angewandt werden.

Unter unzersetzt schmelzbaren Stoffen sind solche Stoffe zu verstehen, welche einen Schmelzpunkt oder Schmelzbereich zwischen 25°C und 300°C, vorzugsweise zwischen 30°C und 150°C und besonders bevorzugt zwischen 35°C und 100°C haben. Als Stoffe kommen dabei sowohl chemisch einheitliche Stoffe als auch Gemische in Betracht

Im Sinne der Erfindung kommen als Stoffe vorzugsweise organische Substanzen in Betracht, und besonders bevorzugt in Wasser schwerlösliche organische Substanzen.

Die erfindungsgemäß einsetzbaren Stoffe sind beispielsweise solche, welche industriellen Einsatz finden in der Kosmetik, der Pharmazie, in Nahrungsmitteln, in Wasch- und Reinigungsmitteln, in Klebstoffen, in der Oberflächenbehandlung, in Hygieneprodukten sowie in der Landwirtschaft. Bevorzugte Stoffe sind die im Bereich der Kosmetik eingesetzten Stoffe wie beispielsweise UV-Lichtschutzfilter, Farbstoffe, Riechstoffe, Emulgatoren, Wachse, Rückfetter, Antioxidantien, Deodorantien und Antitranspirantien. Weiterhin bevorzugte Stoffe sind pharmakologisch aktive Stoffe, die in der Kosmetik, Dermatologie und Pharmazie als Wirkstoffe eingesetzt werden.

Als flüssige Phase kommen im Sinne der Erfindung beispielsweise in Betracht
- Wasser
- niedrige aliphatische Alkohole, z. B. solche mit 1 bis 4 Kohlenstoffatomen, wie Methanol, Ethanol, Isopropylalkohol oder die isomeren Butanole
- Polyole, welche vorzugsweise 2 bis 15 Kohlenstoffatome und mindestens zwei Hydroxylgruppen aufweisen, wie z. B. Ethylenglycol, Diethylenglycol, Propylenglycol, Butylenglycol, Hexylenglycol, Polyethylenglycole mit einem durchschnittlichen Molekulargewicht von 100 bis 1.000 Dalton, Glycerin, technische Oligoglyceringemische mit einem Eigenkondensationsgrad von 1,5 bis 10, wie etwa technische Diglyceringemische mit einem Diglyceringehalt von 40 bis 50 Gew.-%
- Niedrigalkylglucoside, insbesondere solche mit 1 bis 8 Kohlenstoffatomen im Alkylrest wie beispielsweise Methyl- und Butylglucosid
sowie Gemische der vorstehend genannten Stoffe. Als flüssige Phase bevorzugt ist Wasser.

Unter Schwerlöslichkeit ist zu verstehen, daß sich von dem unzersetzt schmelzbaren Stoff in der flüssigen Phase bei 20°C maximal 1 Gew.-%, bevorzugt maximal 0,1 Gew.-% und besonders bevorzugt maximal 0,01 Gew.-% lösen, bezogen auf das Gesamtgewicht der Lösung.

Das Gewichtsverhältnis zwischen dem unzersetzt schmelzbaren Stoff und der flüssigen Phase ist an sich nicht kritisch und ergibt sich im wesentlichen daraus, daß der Stoff in der flüssigen Phase hinreichend verteilt vorliegt.

Unter Oberflächenmodifikationsmitteln sind im Sinne der Erfindung Stoffe zu verstehen, welche der Oberfläche der Nanopartikel physikalisch anhaften, mit diesen jedoch vorzugsweise nicht chemisch reagieren. Die einzelnen an der Oberfläche adsorbierten Moleküle der Oberflächenmodifikationsmittel sind im wesentlichen frei von intermolekularen Bindungen untereinander. Unter Oberflächenmodifikationsmitteln sind insbesondere Dispergiermittel zu verstehen. Dispergiermittel sind dem Fachmann beispielsweise auch unter den Begriffen Emulgatoren, Schutzkolloide, Netzmittel und Detergentien bekannt.

Als Oberflächenmodifikationsmittel kommen beispielsweise Emulgatoren vom Typ der nichtionogenen Tenside aus mindestens einer der folgenden Gruppen in Frage:
(1) Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe;
(2) C_{12/18}-Fettsäuremono- und -diester von Anlagerungsprodukten von 1 bis 30 Mol Ethylenoxid an Glycerin;
(3) Glycerinmono- und -diester und Sorbitanmono- und -diester von gesättigten und ungesättigten Fettsäuren mit 6 bis 22 Kohlenstoffatomen und deren Ethylenoxidanlagerungsprodukte;
(4) Alkylmono- und -oligoglycoside mit 8 bis 22 Kohlenstoffatomen im Alkylrest und deren ethoxylierte Analoga;
(5) Anlagerungsprodukte von 2 bis 60 Mol Ethylenoxid an Ricinusöl und/oder gehärtetes Ricinusöl;
(6) Polyol- und insbesondere Polyglycerinester, wie z.B. Polyglycerinpolyricinoleat, Polyglycerinpoly-12-hydroxystearat oder Polyglycerindimerat. Ebenfalls geeignet sind Gemische von Verbindungen aus mehreren dieser Substanzklassen;
(7) Partialester auf Basis linearer, verzweigter, ungesättigter bzw. gesättigter C_{6/22}-Fettsäuren, Ricinolsäure sowie 12-Hydroxystearinsäure und Glycerin, Polyglycerin, Pentaerythrit, Dipentaerythrit, Zuckeralkohole (z.B. Sorbit), Alkylglucoside (z.B. Methylglucosid, Butylglucosid, Laurylglucosid) sowie Polyglucoside (z.B. Cellulose);
(8) Mono-, Di- und Trialkylphosphate sowie Mono-, Di- und/oder Tri-PEG-alkylphosphate und deren Salze;
(9) Wollwachsalkohole;
(10) Polysiloxan-Polyalkyl-Polyether-Copolymere bzw. entsprechende Derivate;
(11) Mischester aus Pentaerythrit, Fettsäuren, Citronensäure und Fettalkohol gemäß DE-PS 1165574 und/oder Mischester von Fettsäuren mit 6 bis 22 Kohlenstoffatomen, Methylglucose und Polyolen, vorzugsweise Glycerin oder Polyglycerin sowie
(12) Polyalkylenglycole.

Die Anlagerungsprodukte von Ethylenoxid und/oder von Propylenoxid an Fettalkohole, Fettsäuren, Alkylphenole, Glycerinmono- und -diester sowie Sorbitanmonound -diester von Fettsäuren oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/ oder Propylenoxid und Substrat, mit denen die Anlagerungsreaktion durchgeführt wird, entspricht.

C_{8/18}-Alkylmono- und -oligoglycoside, ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 18 C-Atomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

Typische Beispiele für anionische Emulgatoren sind Seifen, Alkylbenzolsulfonate, Alkansulfonate, Olefinsulfonate, Alkylethersulfonate, Glycerinethersulfonate, α-Methylestersulfonate, Sulfofettsäuren, Alkylsulfate, Alkylethersulfate wie beispielsweise Fettalkoholethersulfate, Glycerinethersulfate, Hydroxymischethersulfate, Monoglycerid(ether)sulfate, Fettsäureamid(ether)sulfate, Mono- und Dialkylsulfosuccinate, Mono- und Dialkylsulfo-succinamate, Sulfotriglyceride, Amidseifen, Ethercarbonsäuren und deren Salze, Fettsäureisethionate, Fettsäuresarcosinate, Fettsäuretauride, N-Acylaminosäuren wie beispielsweise Acylglutamate und Acylaspartate, Alkyloligoglucosidsulfate, Proteinfettsäurekondensate (insbesondere pflanzliche Produkte auf Weizenbasis), und Alkyl(ether)phosphate. Sofern die anionischen Tenside Polyglycoletherketten enthalten, können diese eine konventionelle, vorzugsweise jedoch eine eingeengte Homologenverteilung aufweisen.

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammonium-glycinat, N-Acylamino-propyl-N,N-dimethylammoniumglycinate, beispielsweise das Kokosacylaminopropyldimethylammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung Cocamidopropyl Betaine bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C_{12/18}-Acylsarcosin. Neben den ampholytischen kommen auch quartäre Emulgatoren in Betracht, beispielsweise solche vom Typ der Esterquats, vorzugsweise Salze von methylquaternierten Difettsäuretriethanolaminestem.

Als Oberflächenmodifikationsmittel geeignete Schutzkolloide sind z.B. natürliche wasserlösliche Polymere wie z. B. Gelatine, Casein, Gummi arabicum, Lysalbinsäure, Stärke, Albumin, Alginsäure sowie deren Alkali- und Erdalkalimetallsalze, wasserlösliche Derivate von wasserunlöslichen polymeren Naturstoffen wie z. B. Celluloseether wie Methylcellulose, Hydroxyethylcellulose, Carboxymethylcellulose oder modifizierte Carboxymethyl-cellulose, Hydroxyethyl-Stärke oder Hydroxypropyl-Guar, sowie synthetische wasserlösliche Polymere, wie z. B. Polyvinylalkohol, Polyvinylpyrrolidon, Polyalkylenglycole, Polyasparaginsäure und Polyacrylate.

Im Sinne der Erfindung sind bevorzugte Oberflächenmodifikationsmittel die nichtionogenen oder anionischen Tenside und deren Gemische, wobei unter den nichtionogenen Tensiden Anlagerungsprodukte von 2 bis 60 Mol Ethylenoxid an Ricinusöl und/oder gehärtetes Ricinusöl, Alkylmono- und -oligoglykoside mit 8 bis 22 Kohlenstoffatomen im Alkylrest und Polyalkylenglykole besonders bevorzugt sind.

Unter der wirksamen Menge des mindestens einen Oberflächenmodifikationsmittels ist eine solche Menge zu verstehen, die mindestens erforderlich ist, um bei Durchführung des erfindungsgemäßen Verfahrens eine stabile Suspension des unzersetzt schmelzbaren Stoffes zu erhalten. Die erforderliche Menge kann jeweils durch einfache Routineversuche ermittelt werden. In der Regel wird der unzersetzt schmelzbare Stoff und das mindestens eine Oberflächenmodifikationsmittel im Gewichtsverhältnis von 1 : 10 bis 10 : 1 und vorzugsweise von 1 : 2 bis 2 : 1 eingesetzt.

In Schritt (a) des erfindungsgemäßen Verfahrens wird eine Emulsion herstellt aus dem unzersetzt schmelzbaren Stoff, einer flüssigen Phase, in welcher der Stoff schwerlöslich ist, sowie einer wirksamen Menge mindestens eines Oberflächenmodifikationsmittels. In dieser Emulsion liegt der unzersetzt schmelzbare Stoff in flüssiger, d. h. in geschmolzener Form vor. Die Herstellung der Emulsion erfolgt demnach vorzugsweise bei einer Temperatur, die oberhalb des Schmelzpunkts des unzersetzt schmelzbaren Stoffes liegt. Besonders bevorzugt ist eine Temperatur von 5 bis 25 und ganz besonders bevorzugt eine Temperatur von 10 bis 20°C oberhalb des Schmelzpunkts des unzersetzt schmelzbaren Stoffes.

Sofern das Gemisch aus erfindungsgemäß eingesetztem unzersetzt schmelzbaren Stoff und Oberflächenmodifikationsmittel eine Schmelzpunktserniedrigung aufweist, beziehen sich die vorstehenden Angaben auf den Schmelzpunkt des Gemischs anstelle des unzersetzt schmelzbaren Stoffes alleine.

Die Herstellung der Emulsion in Schritt (a) des erfindungsgemäßen Verfahrens kann nach unterschiedlichen Varianten erfolgen. In einer Verfahrensvariante wird der unzersetzt schmelzbare Stoff in der flüssigen Phase vorgelegt, und anschließend wird das Gemisch über den Schmelzpunkt des Stoffes hinaus unter Ausbildung eines Zweiphasensystems erhitzt. Durch Zugabe eines oder mehrerer Oberflächenmodifikationsmittel wird sodann der geschmolzene Stoff emulgiert. In einer anderen Verfahrensvariante werden der Stoff und die flüssige Phase zusammen mit dem oder den Oberflächenmodifikationsmitteln vorgelegt, und dann gemeinsam aufgeschmolzen und emulgiert, oder die zunächst hergestellte Schmelze aus Stoff und Obertlächenmodifikationsmittel(n) wird mit der flüssigen Phase, die gegebenenfalls ebenfalls ein oder mehrere Oberflächenmodifikationsmittel enthält, vermengt. In letzterem Fall ist die Temperatur der flüssigen Phase so zu wählen, daß sie oberhalb des Schmelzpunkts des Gemisches aus Stoff und Oberflächenmodifikationsmittel(n) liegt. In wiederum einer weiteren Verfahrensvariante kann die Schmelze des Stoffes mit einer Lösung eines oder mehrerer Oberflächenmodifikationsmittel in der flüssigen Phase vermengt und emulgiert werden, wobei die Temperatur der Lösung so zu wählen ist, daß sie oberhalb des Schmelzpunkts des Stoffes liegt.

In einer bevorzugten Variante von Schritt (a) des erfindungsgemäßen Verfahrens wird die Emulsion hergestellt, indem der unzersetzt schmelzbare Stoff zusammen mit der flüssigen Phase und dem mindestens einen Oberflächenmodifikationsmittel aufgeschmolzen wird.

In einer weiteren bevorzugten Variante von Schritt (a) des erfindungsgemäßen Verfahrens wird die Emulsion hergestellt, indem der geschmolzene Stoff vorgelegt wird, und anschließend die flüssige Phase und mindestens ein Oberflächenmodifikationsmittel bei einer Temperatur oberhalb des Schmelzpunkts des Stoffes zusammen oder nacheinander zugegeben werden. Im letzteren Fall kann die Zugabe der flüssigen Phase vor oder nach der Zugabe des Oberflächenmodifikationsmittels erfolgen.

Zur Herstellung der Emulsion in Schritt (a) des erfindungsgemäßen Verfahrens erfolgt die Durchmischung der Komponenten vorzugsweise ausschließlich mit einem für technische Rührkessel üblichen Rührer, beispielsweise einem Flügel-, Propeller- oder Impellerrührer.

Die im Schritt (a) des erfindungsgemäßen Verfahrens hergestellte Emulsion wird anschließend im zweiten Schritt des Verfahrens in eine Zone verminderten Druckes so entspannt, daß die Emulsion unter den Schmelzpunkt des Stoffes abgekühlt wird. Für den Fall, daß das in der Emulsion vorliegende Gemisch aus dem Stoff und dem oder den Oberflächenmodifikationsmittel(n) einen niedrigeren Schmelzpunkt hat als der Stoff selbst, muß die Emulsion unter den Schmelzpunkt des Gemischs abgekühlt werden.

Das Einbringen der Emulsion in die Zone verminderten Drucks im Verfahrensschritt (b) geschieht vorzugsweise in der Weise, daß die Abkühlung der Emulsion unter den Schmelzpunkt des Stoffes durch teilweise Verdampfung der flüssigen Phase erfolgt. Der Abkühlungsvorgang erfolgt innerhalb einer sehr kurzen Zeitspanne, die vorzugsweise 0,01 bis 5 Sekunden und besonders bevorzugt 0,1 bis 2 Sekunden beträgt.

Das Vakuum wird so gewählt, daß während der Entspannung durch die stattfindende Verdampfungskühlung eine Verfestigung der emulgierten Teilchen des unzersetzt schmelzbaren Stoffes erfolgt. Die Wahl des geeigneten Vakuums richtet sich somit nach der Art der flüssigen Phase sowie dem Schmelzpunkt des Stoffes bzw. des Gemisches aus dem Stoff und dem oder den Oberflächenmodifikationsmittel(n) und kann vom Fachmann z. B. anhand von Dampfdruckkurven der flüssigen Phase getroffen werden. Im Fall von Wasser als flüssiger Phase liegt ein geeignetes Vakuum im Bereich von von 10 bis 800 mbar. Hier wird beispielsweise bei 200 mbar eine Abkühlung auf ca. 60°C und bei 20 mbar auf ca. 20°C erreicht.

Im Sinne der Erfindung bevorzugt ist im Verfahrensschritt (b) somit ein Druck im Bereich zwischen 10 und 800 mbar und besonders bevorzugt zwischen 20 und 300 mbar.

Die Entspannung der Emulsion ins Vakuum erfolgt in einer Ausführungsform der Erfindung über eine mit einem Ventil versehene Leitung. Alternativ ist auch eine Entspannung über eine Düse möglich.

Überraschenderweise kommt es nach dem erfindungsgemäßen Verfahren zur Bildung nanopartikulärer Suspensionen mit mittleren Teilchendurchmessem im Bereich von 5 bis 500 nm, ohne daß die in Teilschritt (a) des Verfahrens hergestellte Emulsion bereits diese Feinteiligkeit aufweisen muß.

Nach der Lehre des Stands der Technik, wie beispielsweise der eingangs zitierten EP-B1 0 506 197, ist es erforderlich, daß bereits in der zunächst hergestellten Emulsion Teilchen der gewünschten Größe erzeugt werden, was nur durch das Einbringen hoher mechanischer Energie wie z. B. einen Hochdruckhomogenisator möglich ist. Diese Teilchen verfestigen sich anschließend beim Abkühlen, ohne daß es zu einer Veränderung der Teilchengröße, insbesondere einer Verkleinerung der Teilchen, kommt. Ganz im Gegenteil wird in der Literatur wiederholt darauf hingewiesen, daß es bei der Abkühlung nanopartikulärer Emulsionen geschmolzener Stoffe häufig zur Zusammenlagerung und Agglomeration zu gröberen Teilchen kommt.

Im Rahmen der Erfindung wurde demgegenüber festgestellt, daß die zusätzliche Verwendung eines Hochdruckhomogenisators im Teilschritt (a) des erfindungsgemäßen Verfahrens sogar zu einer Verschlechterung führt, d. h. zur Bildung von deutlich gröberen Teilchen.

In dem erfindungsgemäßen Verfahren kommt es somit durch die in Teilschritt (b) gewählte Verfahrensweise gleichzeitig zu einer Zerkleinerung der Partikel des emulgierten Stoffes und einer Verfestigung unter Ausbildung einer nanopartikulären Suspension. Es wird vermutet, daß hierfür die während der Entspannung ins Vakuum auftretenden mechanischen Einflüsse in Verbindung mit der schlagartigen Abkühlung verantwortlich sind, welche die Emulsion im Verlauf von Sekundenbruchteilen in eine Suspension umwandeln.

Nach dem erfindungsgemäßen Verfahren werden nanopartikuläre Suspensionen erhalten, die eine besonders hohe Lagerstabilität selbst unter Temperaturbelastung aufweisen, d. h. weder agglomerieren noch sedimentieren.

Das erfindungsgemäße Herstellverfahren zeichnet sich dadurch aus, daß es lediglich einer einfachen und in vielen Herstellungsbetrieben verbreiteten technischen Infrastruktur bedarf, hohe Raum-Zeit-Ausbeuten liefert, kostengünstig ist und problemlos in größere Maßstäbe übertragen werden kann (scale-up).

Ein weiterer Vorteil des erfindungsgemäßen Verfahrens liegt darin, daß während des Teilschritts (b) infolge der teilweisen Verdampfung der flüssigen Phase eine Aufkonzentrierung der Suspension stattfindet.

Ein weiterer Gegenstand der Erfindung ist somit ein Verfahren wie vorstehend beschrieben, in welchem der Emulsion in Verfahrensschritt (b) 5 bis 50, vorzugsweise aber 10 bis 20 Gew.-% der flüssigen Phase, jeweils bezogen auf das Gesamtgewicht der Emulsion vor der Entspannung, entzogen werden.

Von Vorteil ist diese Aufkonzentrierung, wenn zwar zur Ausbildung der Emulsion im ersten Schritt des Verfahrens eine bestimmte Menge der flüssigen Phase erforderlich ist, diese flüssige Phase aber für die weitere Verwendung der nanopartikulären Suspension störend ist. So ist es für den Einsatz der nanopartikulären Suspensionen beispielsweise in der Kosmetik oder Pharmazie aus unterschiedlichen Gründen, beispielsweise zur Vermeidung von Problemen bei der Formulierung oder zur Vermeidung unerwünschter Begleitstoffe, in der Regel wünschenswert, zusammen mit den Nanopartikeln möglichst wenig Begleitstoffe, wozu auch die flüssige Phase einer Nanopartikel-Suspension zählen kann, in die Formulierung einzubringen.

Falls gewünscht, können die erfindungsgemäß hergestellten nanopartikulären Suspensionen nach an sich bekannten Verfahren, vorzugsweise durch Verdampfung der flüssigen Phase unter reduziertem Druck bei Temperaturen unterhalb des Schmelzpunkts der Nanopartikel, vollständig von der flüssigen Phase befreit werden. Dadurch werden Nanopartikel als fließfähige trockene Pulver erhalten, die selbst nach längerer Lagerung problemlos redispergierbar sind und sich beispielsweise zum Einsatz in der Kosmetik, der Pharmazie, in Nahrungsmitteln, in Wasch- und Reinigungsmitteln, in Klebstoffen, in Hygieneprodukten sowie in der Landwirtschaft eignen.
Die folgenden Beispiele sollen die vorliegende Erfindung verdeutlichen.

### Beispiele

### Beispiel 1: Herstellung einer Suspension von Dehyquart F 75

6,0 kg Dehyquart F 75 (Distearoylethyl Hydroxyethylmonium Methosulfate and Cetearyl Alcohol), 6,0 kg Eumulgin B 2 (Polyoxyethylen-20-Cetylstearylalkohol), und 32,2 kg dest. Wasser wurden in einem Reaktor vorgelegt und auf 60°C erhitzt. Anschließend wurde das Gemisch unter Rühren weiter auf 90-95°C erhitzt, und bei dieser Temperatur eine halbe Stunde nachgerührt. Die entstandene Emulsion wurde über eine Düse innerhalb von 15 Minuten in einen zweiten Reaktor entspannt, der durch Evakuieren auf einem Druck zwischen 20 und 30 mbar gehalten wurde. Während der Entspannung wurde die Dispersion von 90-95°C auf 20-25°C abgekühlt. Es wurden 36,0 kg einer lagerstabilen Suspension erhalten, welche eine mittlere Teilchengröße von 30 nm aufwies.

### Beispiel 2: Herstellung einer Suspension von Dehyquart AU 56

Aus 6,0 kg Dehyquart AU 56 (N-Methyl-triethanolammonium-dialkylestermethosulfat mit 10 Gew.-% Isopropanol), 6,0 kg Plantacare 1200 UP (C12-C16-Alkylglykosid) und 19,8 kg dest. Wasser wurden analog Beispiel 1 28,0 kg Suspension erhalten, welche eine mittlere Teilchengröße von 60 nm aufwies.

### Beispiel 3: Herstellung einer Suspension von Lanette O

Aus 3,0 kg Lanette O (Cetylstearylalkohol), 3,0 kg Texapon N 70 (Natriumlaurylethersulfat mit 2 EO) und 15,6 kg dest. Wasser wurden analog Beispiel 1 umgesetzt, wobei in dem Reaktor, in den entspannt wurde, weitere 12,0 kg Wasser vorgelegt waren. Man erhielt 30,5 kg Suspension, welche eine mittlere Teilchengröße von 200 nm aufwies.

### Beispiel 4: Herstellung einer Suspension von Lanette O

Aus 3,0 kg Lanette O, 3,0 kg Eumulgin HRE 455 (gehärtetes Rizinusöl mit 40 EO in Propylenglykol/Wasser) und 33,1 kg dest. Wasser wurden analog Beispiel 1 32,0 kg Suspension erhalten, welche eine mittlere Teilchengröße von 200 nm aufwies.

### Vergleichsbeispiel 1: Herstellung einer Suspension von Lanette O

Es wurde analog Beispiel 4 verfahren mit dem einzigen Unterschied, daß die Emulsion aus den Einsatzstoffen vor der Entspannung in den zweiten Reaktor dreimal im Kreis durch einen Hochdruckhomogenisator (Cavitron) gefahren wurde. Es wurden 31,5 kg Suspension erhalten, welche eine mittlere Teilchengröße von mehr als 1000 nm aufwies.

## Patentansprüche

1. Verfahren zur Herstellung einer Suspension eines unzersetzt schmelzbaren Stoffes, welcher einen Schmelzpunkt oder Schmelzbereich zwischen 25°C und 300°C aufweist, mit einem mittleren Teilchendurchmesser im Bereich von 5 bis 500 nm, bei dem man
(a) eine Emulsion herstellt aus dem unzersetzt schmelzbaren Stoff, einer flüssigen Phase, in welcher der Stoff schwerlöslich ist, sowie einer wirksamen Menge mindestens eines Oberflächenmodifikationsmittels, und
(b) die resultierende Emulsion in eine Zone verminderten Druckes so entspannt, daß die Emulsion unter den Schmelzpunkt des Stoffes abgekühlt wird.

2. Verfahren nach Anspruch 1, **dadurch gekennzeichnet, daß** der mittlere Teilchendurchmesser im Bereich von 10 bis 300 nm liegt.

3. Verfahren nach einem der Ansprüche 1 oder 2, **dadurch gekennzeichnet, daß** die Emulsion aus Verfahrensschritt (a) hergestellt wird, indem der Stoff zusammen mit der flüssigen Phase und dem mindestens einen Oberflächenmodifikationsmittel aufgeschmolzen wird.

4. Verfahren nach einem der Ansprüche 1 oder 2, **dadurch gekennzeichnet, daß** die Emulsion aus Verfahrensschritt (a) hergestellt wird, indem der geschmolzene Stoff vorgelegt wird, und anschließend die flüssige Phase und mindestens ein Oberflächenmodifikationsmittel zusammen oder nacheinander zugegeben werden.

5. Verfahren nach einem der Ansprüche 1 bis 4, **dadurch gekennzeichnet, daß** in Verfahrensschritt (b) die Abkühlung der Emulsion unter den Schmelzpunkt des Stoffes durch teilweise Verdampfung der flüssigen Phase erfolgt.

6. Verfahren nach einem der Ansprüche 1 bis 5, **dadurch gekennzeichnet, daß** in Verfahrensschritt (b) der Druck im Bereich zwischen 10 und 800 mbar, vorzugsweise zwischen 20 und 300 mbar liegt.

7. Verfahren nach einem der Ansprüche 1 bis 6, **dadurch gekennzeichnet, daß** der Emulsion in Verfahrensschritt (b) 5 bis 50, vorzugsweise 10 bis 20 Gew.-% der flüssigen Phase, jeweils bezogen auf das Gesamtgewicht der Emulsion vor der Entspannung, entzogen werden.

## Claims

1. A process for the production of a suspension of a substance which can be melted without decomposing and which has a melting point or melting range of 25 to 300°C and a mean particle diameter in the range from 5 to 500 nm, **characterized in that**
(a) an emulsion is prepared from the substance which can be melted without decomposing, a liquid phase in which the substance is poorly soluble and an effective quantity of at least one surface modifier and
(b) the resulting emulsion is expanded into a zone of reduced pressure so that the emulsion is cooled to below the melting point of the substance.

2. A process as claimed in claim 1, **characterized in that** the mean particle diameter is in the range from 10 to 300 nm.

3. A process as claimed in claim 1 or 2, **characterized in that** the emulsion from step (a) is prepared by melting the substance together with the liquid phase and the at least one surface modifier.

4. A process as claimed in claim 1 or 2, **characterized in that** the emulsion from step (a) is prepared by initially introducing the molten substance and then adding the liquid phase and at least one surface modifier either together or successively.

5. A process as claimed in any of claims 1 to 4, **characterized in that** the cooling of the emulsion to below the melting point of the substance in step (c) is effected by partial evaporation of the liquid phase.

6. A process as claimed in any of claims 1 to 5, **characterized in that** the pressure in step (b) is in the range from 10 to 800 mbar and preferably in the range from 20 to 300 mbar.

7. A process as claimed in any of claims 1 to 6, **characterized in that** 5 to 50% by weight and preferably 10 to 20% by weight of the liquid phase is removed from the emulsion in step (b), based on the total weight of the emulsion before expansion.

## Revendications

1. Procédé pour la préparation d'une suspension d'une substance fusible sans décomposition, qui présente un point de fusion ou une plage de fusion entre 25°C et 300°C, avec un diamètre moyen de particules dans la plage de 5 à 500 nm, dans lequel
(a) on prépare une émulsion de la substance fusible sans décomposition, une phase liquide dans laquelle la substance est peu soluble ainsi qu'une quantité efficace d'au moins un agent de modification de surface et
(b) on détend l'émulsion obtenue dans une zone de pression réduite de telle manière que l'émulsion est refroidie au-dessous du point de fusion de la substance.

2. Procédé selon la revendication 1, **caractérisé en ce que** le diamètre moyen des particules est situé dans la plage de 10 à 300 nm.

3. Procédé selon l'une quelconque des revendications 1 ou 2, **caractérisé en ce que** l'émulsion de l'étape de procédé (a) est préparée **en ce qu'**on fond la substance avec la phase liquide et au moins un agent de modification de surface.

4. Procédé selon l'une quelconque des revendications 1 ou 2, **caractérisé en ce que** l'émulsion de l'étape de procédé (a) est préparée **en ce qu'**on dispose au préalable la substance fondue et on ajoute ensuite, ensemble ou consécutivement, la phase liquide et au moins un agent de modification de surface.

5. Procédé selon l'une quelconque des revendications 1 à 4, **caractérisé en ce que** dans l'étape de procédé (b) le refroidissement de l'émulsion au-dessous du point de fusion de la substance est réalisé par évaporation partielle de la phase liquide.

6. Procédé selon l'une quelconque des revendications 1 à 5, **caractérisé en ce que** dans l'étape de procédé (b), la pression se situe dans la plage entre 10 et 800 mbars, de préférence entre 20 et 300 mbars.

7. Procédé selon l'une quelconque des revendications 1 à 6, **caractérisé en ce qu'**on soutire de l'émulsion dans l'étape de procédé (b), avant la détente, 5 à 50, de préférence 10 à 20% en poids de la phase liquide, à chaque fois par rapport au poids total de l'émulsion.
